# EUROPEAN PATENT APPLICATION

(11) **EP 4 487 895 A1**
(43) Date of publication of application: **08.01.2025**
(21) Application number: 23774469.3
(22) Date of filing: 06.03.2023
(51) Int. Cl.: A61M 25/06

(54) **CATHETER ASSEMBLY**

(30) Priority: 22.03.2022 JP 2022044898
(71) Applicant: Terumo Kabushiki Kaisha, Tokyo 151-0072 (JP)
(72) Inventor: ISHIDA, Masahiro, Ashigarakami-gun, Kanagawa 259-0151 (JP)
(74) Representative: TBK
(86) International application number: PCT/JP2023/008209
(87) International publication number: WO 2023/181870

(57) **Abstract**

In a catheter assembly (10), a connection portion (46) of a catheter operation member (20) includes a first restriction portion (82). A safety member (22) includes a second restriction portion (102) located on the distal end side with respect to a first restriction portion (82) so as to face a first restriction portion (82) in an initial state. When the safety member (22) is relatively moved with respect to the catheter operation member (20) in a proximal end direction, the catheter operation member (20) and the safety member (22) are separated from each other since the connection portion (46) pressed by a second restriction portion (102) is elastically deformed and the second restriction portion (102) passes over the first restriction portion (82) in the proximal end direction.

## Description

### Technical Field

The present invention relates to a catheter assembly.

### Background Art

WO 2018/038029 A discloses a catheter assembly including a catheter operation member for moving a catheter member in a distal end direction with respect to a needle member and a safety member for covering a needle tip. The safety member includes a pair of arms separably connected to a valve mechanism inserted into a lumen of a catheter hub, and an outer tube covering a pair of the arms. In an initial state of the catheter assembly, a pair of the arms are hooked on the valve mechanism, and the outer tube covers a pair of the arms so as to prevent a pair of the arms from opening outward. When the catheter assembly is used, a pair of the arms are exposed from the outer tube and opened outward to be disengaged from the valve mechanism.

### Summary of Invention

In the catheter assembly disclosed in WO 2018/038029 A, the safety member is configured by assembling a plurality of members in a complicated manner. Therefore, there is a problem that the size of the catheter assembly increases and the manufacturing cost tends to increase.

An object of the present invention is to solve the above-described problem.

According to an aspect of the present invention, there is provided a catheter assembly including: a catheter member including a catheter and a catheter hub provided at a proximal end of the catheter; a needle member including an inner needle inserted into a lumen of the catheter and an inner needle hub provided at a proximal end of the inner needle; a catheter operation member provided in the catheter member; and a safety member provided to be movable with respect to the inner needle in an axial direction of the inner needle, in which a needle tip of the inner needle is exposed from the safety member in an initial state, is located on a distal end side with respect to the safety member, and is covered with the safety member in a state where the inner needle is removed from the catheter member, the catheter operation member includes a connection portion that separably connects the safety member and is formed to be elastically deformable, the connection portion includes a first restriction portion, the safety member includes a second restriction portion located on a distal end side with respect to the first restriction portion so as to face the first restriction portion in the initial state, and when the safety member is relatively moved to the catheter operation member in a proximal end direction, the catheter operation member and the safety member are separated from each other since the connection portion pressed by the second restriction portion is elastically deformed and the second restriction portion passes over the first restriction portion in the proximal end direction.

According to the present invention, when the catheter operation member is moved in the distal end direction to advance the catheter in a state where the living body is punctured with the inner needle, the second restriction portion of the safety member can be pushed in the distal end direction by the first restriction portion of the catheter operation member. Thus, the safety member can be moved in the distal end direction along with the operation of the catheter operation member.

Furthermore, when the safety member is pulled back in the proximal end direction from the catheter operation member after the catheter is advanced, the first restriction portion is pressed in the proximal end direction by the second restriction portion, and thus the connection portion can be elastically deformed. Thus, the safety member can be separated from the catheter operation member by causing the second restriction portion to pass over the first restriction portion in the proximal end direction. Therefore, the needle member can be removed from the catheter member while protecting the needle tip by using the safety member.

In this case, since the first restriction portion is provided in the connection portion of the catheter operation member and the second restriction portion is only required to be provided in the safety member, the configurations of the catheter operation member and the safety member can be simplified. Therefore, the size of the catheter assembly can be reduced and the manufacturing cost can be suppressed.

### Brief Description of Drawings

Fig. 1 is a perspective view of a catheter assembly according to an embodiment of the present invention.
Fig. 2 is an exploded perspective view of the catheter assembly of Fig. 1.
Fig. 3 is an exploded perspective view of an assembly main body of Fig. 2.
Fig. 4 is a partially omitted perspective view of the assembly main body of Fig. 2 as viewed obliquely from below.
Fig. 5 is a partially omitted bottom view of the assembly main body of Fig. 2.
Fig. 6 is a longitudinal cross-sectional view taken along line VI-VI of Fig. 5.
Fig. 7 is a transverse cross-sectional view taken along line VII-VII of Fig. 6.
Fig. 8 is a perspective view of a safety member in Fig. 3 as viewed obliquely from below.
Fig. 9 is an explanatory diagram of a method of assembling a safety member to a catheter operation member.
Fig. 10 is a first explanatory diagram of an operation procedure of the catheter assembly of Fig. 1.
Fig. 11 is a second explanatory diagram of an operation procedure of the catheter assembly of Fig. 1.
Fig. 12 is a third explanatory diagram of an operation procedure of the catheter assembly of Fig. 1.
Fig. 13 is a fourth explanatory diagram of an operation procedure of the catheter assembly of Fig. 1.
Fig. 14 is a fifth explanatory diagram of an operation procedure of the catheter assembly of Fig. 1.
Fig. 15 is a partially omitted longitudinal cross-sectional view of an assembly main body according to a modification example.
Fig. 16 is a transverse cross-sectional view taken along line XVI-XVI of Fig. 15.

### Description of Embodiments

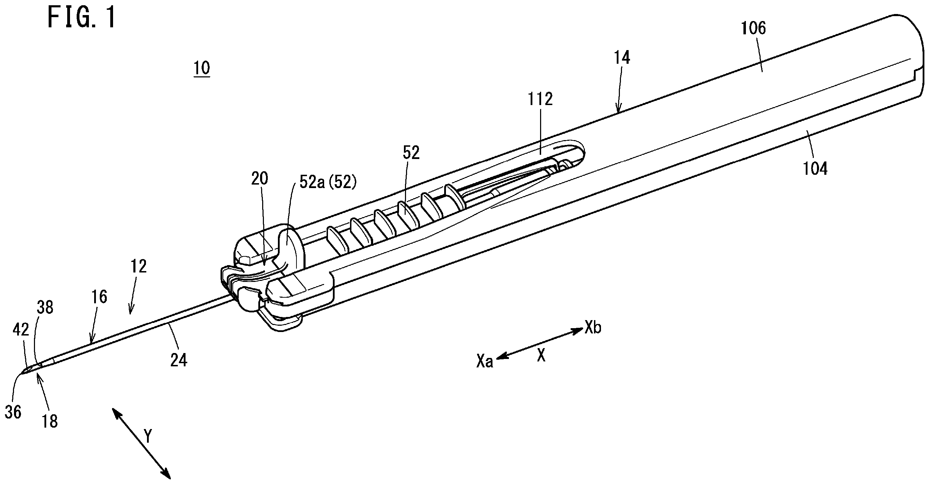

As illustrated in Fig. 1, a catheter assembly 10 according to an embodiment of the present invention is used in a case where infusion, blood transfusion, blood sampling, or the like is performed on a living body. In the catheter assembly 10, a catheter 24 is inserted into and indwelled in a body to connect the inside of the body with the outside of the body. The catheter assembly 10 can insert a catheter longer than a peripheral venous catheter (for example, a central venous catheter, a PICC, a midline catheter, or the like). Note that the catheter assembly 10 may be configured to allow insertion of the peripheral venous catheter. Furthermore, the catheter assembly 10 is not limited to the venous catheter, and may be configured to allow insertion of an artery catheter such as a peripheral arterial catheter.

The catheter assembly 10 extends in one direction (arrow X direction). The catheter assembly 10 has a distal end at one end (arrow Xa direction) in an extending direction. The catheter assembly 10 has a proximal end at the other end (arrow Xb direction) in the extending direction. In the following description, the arrow Xa direction may be referred to as a "distal end direction", and the arrow Xb direction may be referred to as a "proximal end direction". Hereinafter, the configuration of the catheter assembly 10 in the initial state will be described.

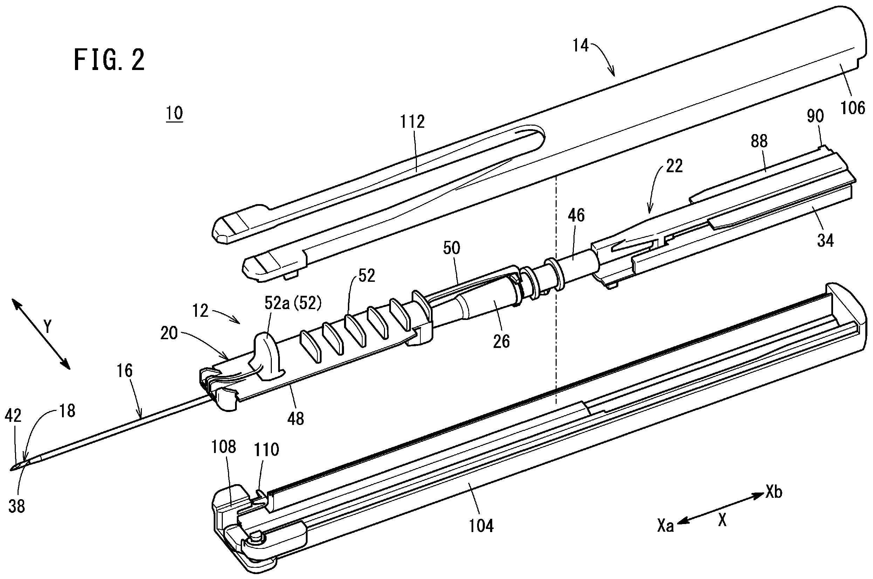

In Fig. 2, the catheter assembly 10 includes an assembly main body 12 and a grip 14. The assembly main body 12 includes a catheter member 16, a needle member 18, a catheter operation member 20, and a safety member 22.

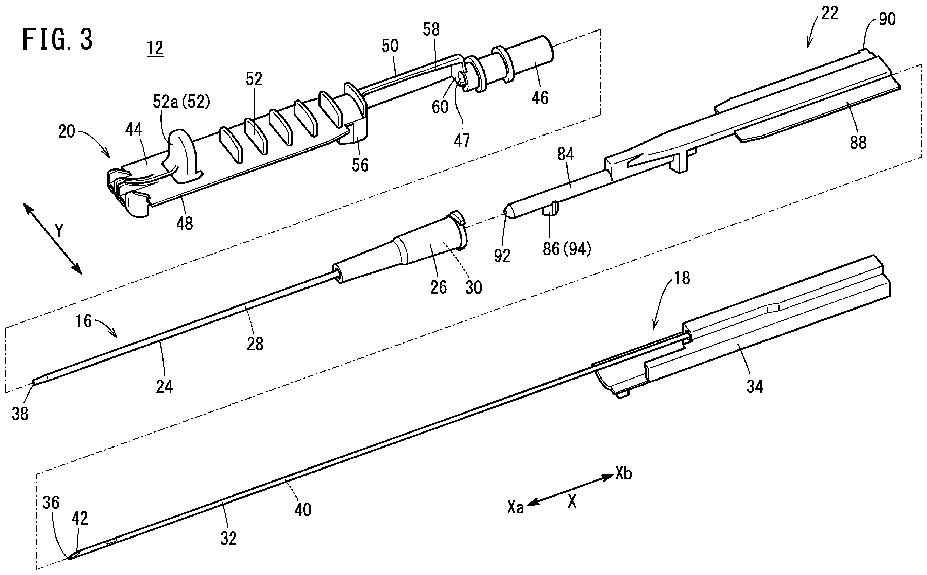

As illustrated in Fig. 3, the catheter member 16 includes a catheter 24 and a catheter hub 26. The catheter 24 has flexibility. The catheter 24 has a lumen 28 having a size capable of accommodating an inner needle 32 of the needle member 18 and allowing a medicinal solution, blood, or the like to flow. The distal end (end portion in the arrow Xa direction) of the catheter 24 is reduced in diameter in order to reduce puncture resistance.

The entire length of the catheter 24 is not particularly limited, and can be appropriately designed according to use, various conditions, and the like, and is set to, for example, about 14 mm to about 500 mm. Furthermore, the entire length of the catheter 24 may be set to about 30 mm to about 400 mm. Moreover, the entire length of the catheter 24 may be set to about 76 mm to about 200 mm. The proximal end (end portion in the arrow Xb direction) of the catheter 24 is fixed to the catheter hub 26 by caulking, fusion, adhesion, or the like.

The constituent material of the catheter 24 is not particularly limited, but a soft resin material is preferable. Specifically, examples of the constituent material of the catheter 24 include a fluorine-based resin such as polytetrafluoroethylene (PTFE), an ethylene-tetrafluoroethylene copolymer (ETFE), and a perfluoroalkoxy fluorine resin (PFA), an olefin-based resin such as polyethylene and polypropylene or a mixture thereof, polyurethane, polyester, polyamide, a polyether nylon resin, and a mixture of the olefin-based resin and an ethylene-vinyl acetate copolymer.

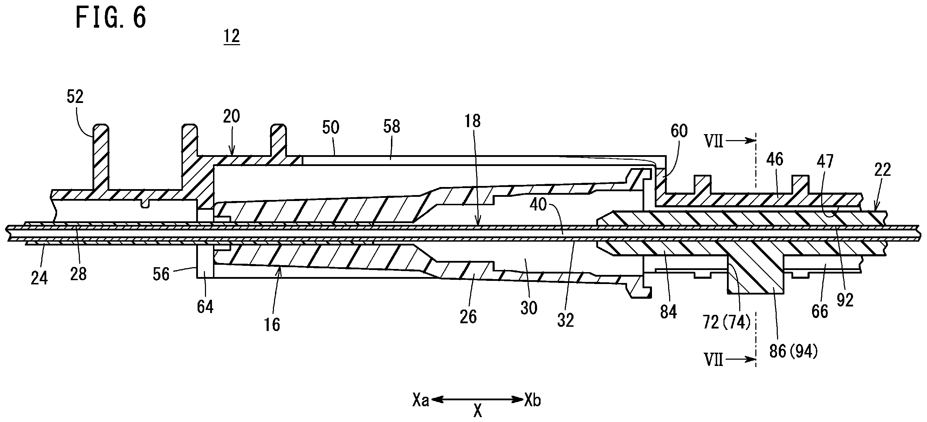

In Fig. 6, the catheter hub 26 includes a lumen 30 communicating with the lumen 28 of the catheter 24. The catheter hub 26 has a tubular shape. A hemostasis valve and a plug (not illustrated) are accommodated in the lumen 30 of the catheter hub 26.

The constituent material of the catheter hub 26 is not particularly limited, but a thermoplastic resin is preferable. Specifically, examples of the constituent material of the catheter hub 26 include polypropylene, polycarbonate, polyamide, polysulfone, polyarylate, and a methacrylate-butylene-styrene copolymer.

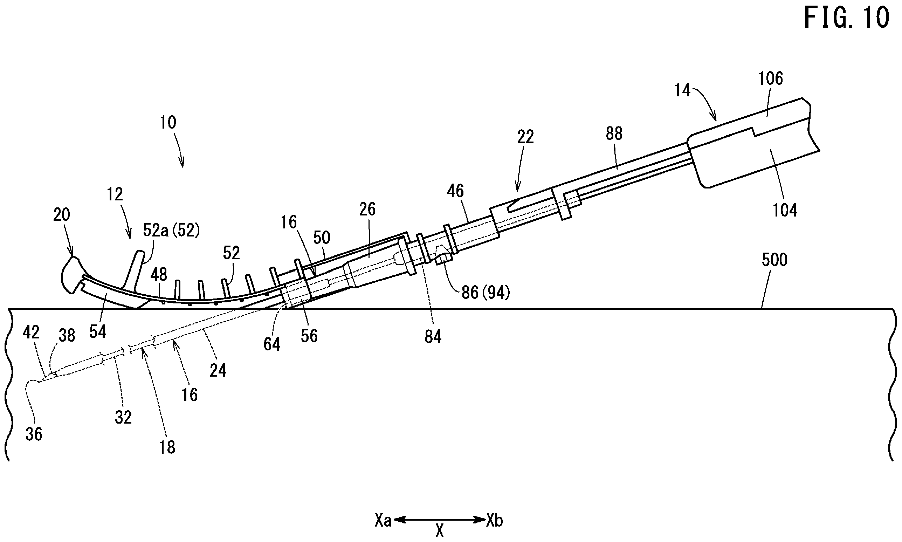

As illustrated in Fig. 3, the needle member 18 includes an inner needle 32 and an inner needle hub 34. The inner needle 32 is a hollow needle having rigidity, which is capable of puncturing a skin 500 (see Fig. 10) of the living body. The entire length of the inner needle 32 is longer than the entire length of the catheter 24. The distal end of the inner needle 32 has a sharp needle tip 36. The inner needle 32 is inserted into the lumen 28 of the catheter 24. The distal end of the inner needle 32 is exposed from the distal end opening 38 of the catheter 24 (see Figs. 1 and 2).

In Fig. 6, the inner needle 32 has a lumen 40 extending along an axial direction of the inner needle 32. The lumen 40 communicates with a distal end opening 42 of the inner needle 32 (see Fig. 3). Examples of the constituent material of the inner needle 32 include a metal material, a hard resin, and ceramics. Examples of the metal material include stainless steel, aluminum, and titanium. The proximal end of the inner needle 32 is fixed to the inner needle hub 34 by fusion, adhesion, insert molding, or the like. The inner needle 32 may be a solid needle.

The inner needle hub 34 extends along the axial direction of the inner needle 32. The inner needle hub 34 is made of, for example, the same material as that of the above-described catheter hub 26. The inner needle hub 34 is fixed to the grip 14 (see Fig. 2).

As illustrated in Figs. 2 to 4, the catheter operation member 20 is operated by a user when the catheter member 16 is moved along the inner needle 32. The catheter operation member 20 covers at least a part of the catheter 24 from above. The catheter operation member 20 is an integrally molded article made of a resin material. The catheter operation member 20 has flexibility. In other words, the catheter operation member 20 is elastically deformable.

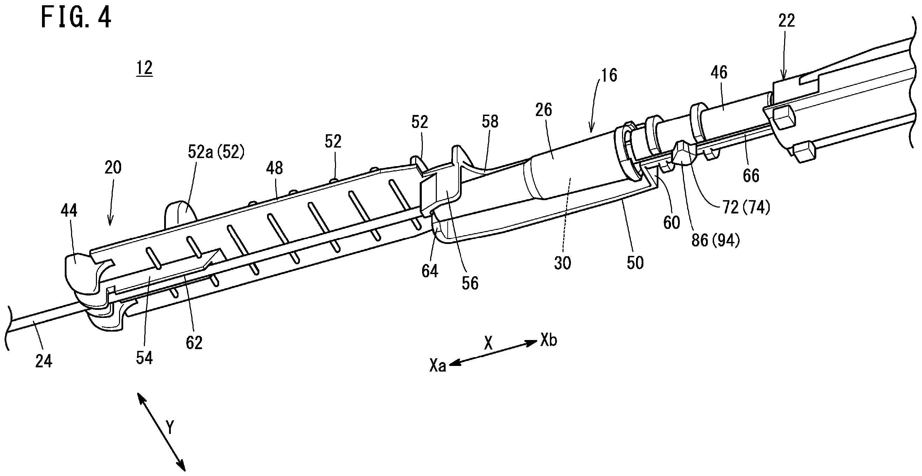

In Figs. 3 and 4, the catheter operation member 20 includes a catheter attachment portion 44 and a connection portion 46. The catheter attachment portion 44 can be detachably attached to the catheter member 16. The catheter attachment portion 44 extends along the axial direction (arrow X direction) of the catheter 24. The entire length of the catheter attachment portion 44 is shorter than the entire length of the catheter member 16 (see Fig. 2).

The catheter attachment portion 44 includes an operation plate portion 48, a cover portion 50, a plurality of operation tabs 52, a first support portion 54, and a second support portion 56. The operation plate portion 48 is located above the catheter 24. The operation plate portion 48 has flexibility. In other words, the operation plate portion 48 is configured to be bendable in a thickness direction of the operation plate portion 48. The width of the operation plate portion 48 is wider than the outer diameter of the catheter 24.

The cover portion 50 extends from the proximal end of the operation plate portion 48 in the proximal end direction (arrow Xb direction). The cover portion 50 is opened downward. The distal end of the cover portion 50 has a cross-sectional shape in which a U shape is vertically inverted. A portion of the cover portion 50 from the intermediate portion to the proximal end in the extending direction has a transverse cross-sectional shape in which an L shape is vertically inverted. In other words, the portion of the cover portion 50 from the intermediate portion to the proximal end includes a cutout portion 58.

That is, a part of the catheter hub 26 is exposed upward from the cover portion 50 (see Fig. 2). That is, the cover portion 50 covers only a part of the catheter hub 26. In Fig. 4, the proximal end of the cover portion 50 is located on the proximal end side (arrow Xb direction) with respect to the proximal end of the catheter hub 26. The proximal end of the cover portion 50 has a wall portion 60 that covers the catheter hub 26 from the proximal end direction.

In Fig. 3, a plurality of the operation tabs 52 protrude upward from the upper surface of the operation plate portion 48. A plurality of the operation tabs 52 are disposed at intervals along the extending direction of the operation plate portion 48. Among a plurality of the operation tabs 52, an operation tab 52a located closest to the arrow Xa direction protrudes more than a plurality of the other operation tabs 52.

In Fig. 4, the first support portion 54 protrudes downward from the lower surface of the distal end of the operation plate portion 48. The first support portion 54 extends along the extending direction of the operation plate portion 48. The first support portion 54 has a first support slit 62 opened downward. The first support slit 62 extends along the extending direction (arrow X direction) of the operation plate portion 48. An intermediate portion of the catheter 24 in the longitudinal direction is detachably fitted to the first support slit 62. In other words, the intermediate portion of the catheter 24 in the longitudinal direction is detachably inserted into the first support slit 62. Note that, in the inserted state, a gap may be present between the intermediate portion of the catheter 24 in the longitudinal direction and the inner surface of the first support slit 62.

The second support portion 56 is located at the distal end of the cover portion 50. The second support portion 56 has a second support slit 64 opened downward. The proximal end of the catheter 24 is detachably fitted to the second support slit 64. In other words, the proximal end of the catheter 24 is detachably inserted into the second support slit 64. Note that, in the inserted state, a gap may be present between the proximal end of the catheter 24 and the inner surface of the second support slit 64.

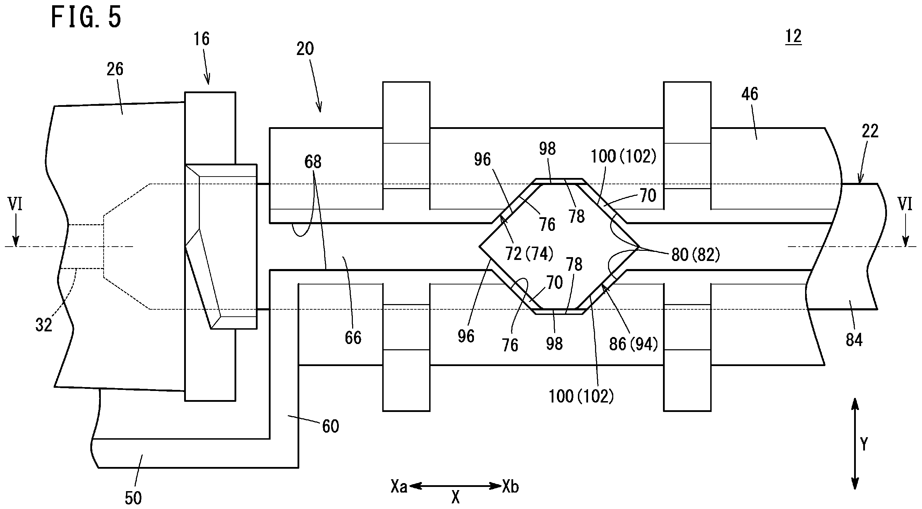

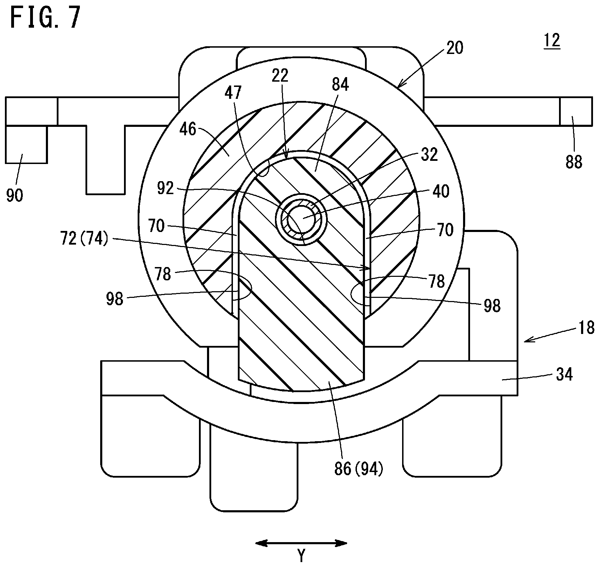

In Figs. 3 to 6, the connection portion 46 extends in the proximal end direction from the proximal end (wall portion 60) of the catheter attachment portion 44. The safety member 22 is separably connected to the connection portion 46. The connection portion 46 has a tubular shape. In Fig. 5, the connection portion 46 is formed with a slit 66 extending along the axial direction (arrow X direction) of the connection portion 46. That is, the connection portion 46 has a C-shape in cross section (see Fig. 7). The slit 66 is provided on the lower surface of the connection portion 46.

The slit 66 extends linearly over the entire length of the connection portion 46. The groove width of the slit 66 is larger than the outer diameter of the inner needle 32 and smaller than the outer diameter of a needle protection portion 84 of the safety member 22, the needle protection portion 84 being to be described later. A recess 70 is formed in each of a pair of groove inner surfaces 68 forming the slit 66.

A pair of the recesses 70 face each other with the slit 66 interposed therebetween. The portion of the slit 66 interposed between a pair of the recesses 70 and a pair of the recesses 70 constitute a first fitting portion 72 for connecting the safety member 22. The first fitting portion 72 is a fitting hole 74 provided in the connection portion 46. The first fitting portion 72 is located on the midway of the connection portion 46 in the axial direction. Specifically, the first fitting portion 72 is located on the distal end side with respect to the center of the connection portion 46 in the axial direction.

The recess 70 is formed in a trapezoidal shape in a bottom view of the connection portion 46. The length of the recess 70 along the arrow X direction decreases outward in the width direction of the slit 66. The recess 70 is surrounded by a first inclined inner surface 76, an intermediate inner surface 78, and a second inclined inner surface 80. Each of the first inclined inner surface 76, the intermediate inner surface 78, and the second inclined inner surface 80 is a flat surface.

The first inclined inner surface 76 is connected to a portion of the groove inner surface 68 located on the distal end side with respect to the recess 70. The first inclined inner surface 76 is inclined outward in the width direction of the slit 66 toward the proximal end (arrow Xb direction) of the connection portion 46. The intermediate inner surface 78 is connected to an end of the first inclined inner surface 76 in the arrow Xb direction. The intermediate inner surface 78 extends along the axial direction of the connection portion 46.

The second inclined inner surface 80 is connected to an end of the intermediate inner surface 78 in the arrow Xb direction. The second inclined inner surface 80 is inclined inward in the width direction of the slit 66 toward the proximal end (arrow Xb direction) of the connection portion 46. The second inclined inner surface 80 is connected to a portion of the groove inner surface 68 located on the proximal end side with respect to the recess 70. The second inclined inner surface 80 is a first restriction portion 82 for restricting the movement of the safety member 22 in the proximal end direction with respect to the connection portion 46.

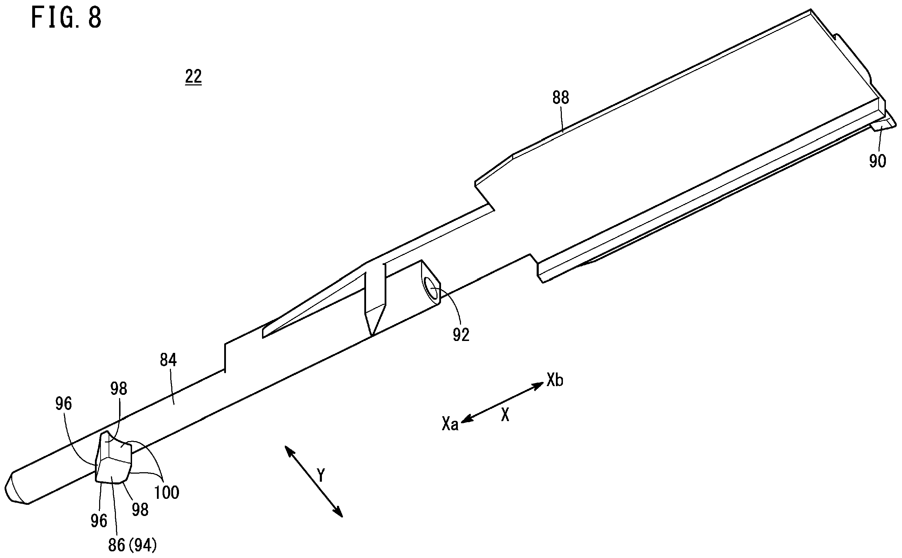

As illustrated in Fig. 8, the safety member 22 includes a needle protection portion 84, a second fitting portion 86, a support plate portion 88, and a stopper portion 90. The needle protection portion 84 is a tubular portion extending along the arrow X direction. That is, the needle protection portion 84 has a lumen 92 into which the inner needle 32 is inserted. The lumen 92 extends over the entire length of the needle protection portion 84. The needle protection portion 84 is inserted into a lumen 47 of the connection portion 46 (see Figs. 6 and 7). In the initial state of the catheter assembly 10, the distal end of the needle protection portion 84 is located in the lumen 30 of the catheter hub 26 (see Fig. 6).

The second fitting portion 86 is a fitting protrusion 94 protruding downward from the lower surface of the needle protection portion 84. The second fitting portion 86 is located on the distal end side with respect to the center of the needle protection portion 84 in a longitudinal direction. In the initial state of the catheter assembly 10, the second fitting portion 86 (fitting protrusion 94) is fitted to the first fitting portion 72 (fitting hole 74) (see Figs. 4 and 5). The second fitting portion 86 has a shape corresponding to the first fitting portion 72. The second fitting portion 86 is formed in a hexagonal shape when viewed from below.

As illustrated in Fig. 5, the outer peripheral surface of the second fitting portion 86 includes a pair of first inclined outer surfaces 96, a pair of intermediate outer surfaces 98, and a pair of second inclined outer surfaces 100. A pair of the first inclined outer surfaces 96, a pair of the intermediate outer surfaces 98, and a pair of the second inclined outer surfaces 100 are flat surfaces.

Each of the first inclined outer surfaces 96 is inclined outward in the width direction (arrow Y direction) of the second fitting portion 86 from the end of the second fitting portion 86 in the arrow Xa direction toward the arrow Xb direction. Each of the intermediate outer surfaces 98 is connected to an end of the first inclined outer surfaces 96 in the arrow Xb direction. The intermediate outer surface 98 extends along the axial direction of the needle protection portion 84. Each of the second inclined outer surfaces 100 is inclined inward in the width direction of the second fitting portion 86 from the proximal end of the intermediate outer surface 98 toward the arrow Xb direction.

In the initial state of the catheter assembly 10, the first inclined outer surface 96 is located to face the first inclined inner surface 76, the intermediate outer surface 98 is located to face the intermediate inner surface 78, and the second inclined outer surface 100 is located to face the second inclined inner surface 80. The second inclined outer surface 100 is a second restriction portion 102 that can come into contact with the second inclined inner surface 80 (first restriction portion 82) in a state where the safety member 22 is connected to the catheter operation member 20. The second restriction portion 102 is located on the distal end side with respect to the first restriction portion 82 in the initial state of the catheter assembly 10.

In Fig. 8. the support plate portion 88 extends in the proximal end direction (arrow Xb direction) from the proximal end of the needle protection portion 84. In Figs. 7 and 8, the stopper portion 90 protrudes downward from the proximal end of the support plate portion 88. The stopper portion 90 is located at an end of the support plate portion 88 in the arrow Y direction. The surface of the stopper portion 90 facing the arrow Xa direction has a portion inclined downward in the arrow Xb direction.

In Fig. 2, the grip 14 has a size and a shape, with which the user can easily grip the grip 14. The grip 14 extends in the arrow X direction. The grip 14 covers the proximal end of the assembly main body 12. The grip 14 includes a lower cover portion 104 and an upper cover portion 106.

The inner needle hub 34 is fixed to the lower cover portion 104. The safety member 22 is provided on the lower cover portion 104 so as to be slidable in the arrow X direction. A movement restriction portion 108 and a holding portion 110 are provided at the distal end of the lower cover portion 104.

The movement restriction portion 108 is a wall portion located on the distal end side with respect to the stopper portion 90 of the safety member 22. When the stopper portion 90 comes into contact with the movement restriction portion 108, the movement of the safety member 22 in the distal end direction with respect to the grip 14 is prevented. The holding portion 110 is located away from the movement restriction portion 108 in the arrow Xb direction. The holding portion 110 is configured such that the stopper portion 90 can pass over in the distal end direction but cannot pass over in the proximal end direction. The holding portion 110 prevents the movement of the safety member 22 in the proximal end direction with respect to the grip 14.

As illustrated in Fig. 2, an opening portion 112 for exposing the catheter operation member 20 is formed in the upper cover portion 106. A plurality of the operation tabs 52 are exposed to the outside from the opening portion 112 of the upper cover portion 106. The grip 14 supports the operation plate portion 48 of the catheter operation member 20 so as to be slidable in the arrow X direction.

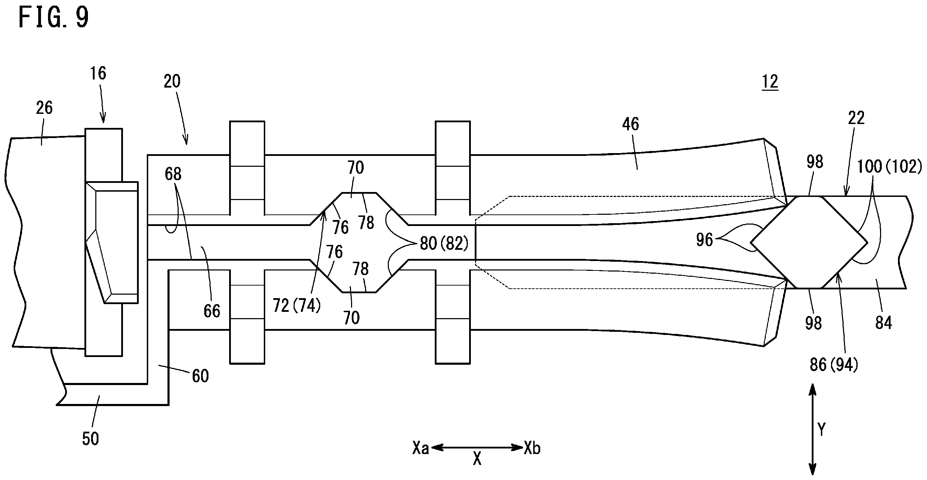

Next, a method of assembling the safety member 22 to the catheter operation member 20 will be described. As illustrated in Fig. 9, the distal end of the safety member 22 is inserted into the lumen 47 of the connection portion 46 from the proximal end side of the catheter operation member 20.

Then, since the groove inner surfaces 68 of the slit 66 are pressed outward in the width direction of the slit 66 by the second fitting portion 86, the groove width of the portion of the slit 66 located on the arrow Xb direction side with respect to the first fitting portion 72 is widened. Therefore, the second fitting portion 86 can be pushed into the first fitting portion 72 (fitting hole 74) through the slit 66.

When the second fitting portion 86 is fitted to the first fitting portion 72, the pressing force acting on the groove inner surface 68 is released. Therefore, the portion of the slit 66 located on the arrow Xb direction side with respect to the first fitting portion 72 returns to the original shape. Thus, the assembly of the safety member 22 to the catheter operation member 20 is completed.

Next, an operation procedure of the catheter assembly 10 will be briefly described. In the case of using the catheter assembly 10, as illustrated in Fig. 10, the skin 500 is punctured with the distal end (the inner needle 32 and the catheter 24) of the catheter assembly 10. At this time, the needle tip 36 of the inner needle 32 is located in the blood vessel.

Next, the user grips the operation tab 52 with fingers and moves the catheter operation member 20 toward the distal end of the catheter 24. Then, the catheter member 16 moves in the distal end direction along the inner needle 32. At this time, since the second inclined inner surface 80 (first restriction portion 82) of the catheter operation member 20 pushes the second inclined outer surface 100 (second restriction portion 102) of the safety member 22 in the distal end direction, the safety member 22 moves in the distal end direction together with the catheter operation member 20.

Note that, at this time, since the safety member 22 is movable in the distal end direction with respect to the grip 14, a large force that causes the second fitting portion 86 to come out from the first fitting portion 72 in the proximal end direction does not act on the connection portion 46. That is, the connection between the catheter operation member 20 and the safety member 22 is maintained in a state where the second inclined outer surface 100 and the second inclined inner surface 80 are brought into contact with each other.

Furthermore, the user separates the catheter 24 from the first support slit 62 by curving the operation plate portion 48 upward while moving the catheter operation member 20. Note that the operation plate portion 48 is located on the skin 500 in a curved state. Thus, the catheter operation member 20 is detached from the grip 14. Therefore, the catheter 24 is indwelled in the blood vessel, and the catheter operation member 20 is detached from the grip 14.

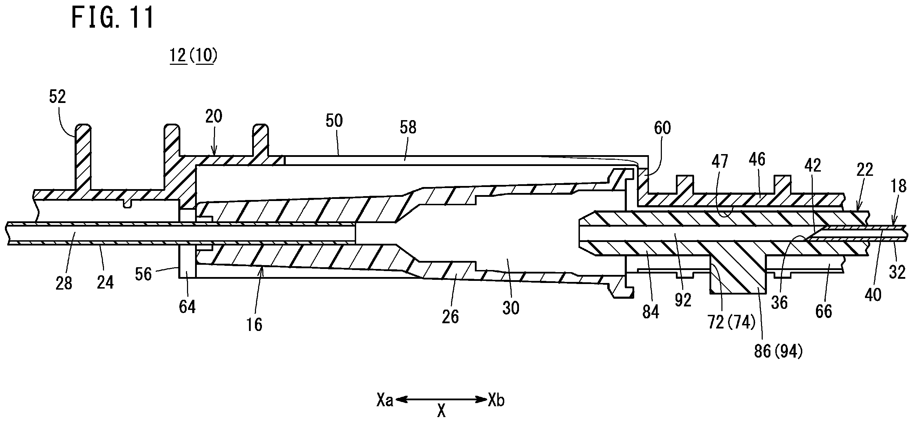

Subsequently, the user pulls back the grip 14 in the proximal end direction (arrow Xb direction) while holding the catheter operation member 20. Then, since the inner needle 32 moves in the proximal end direction together with the grip 14 with respect to the catheter member 16, the needle tip 36 is located in the lumen 92 of the needle protection portion 84 of the safety member 22 (see Fig. 11) .

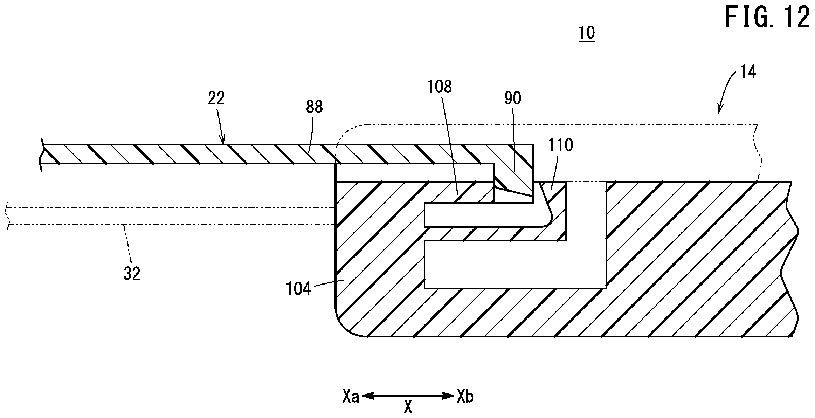

Furthermore, in this state, as illustrated in Fig. 12, the stopper portion 90 of the safety member 22 passes over the holding portion 110 in the distal end direction and comes into contact with the movement restriction portion 108, such that the movement of the safety member 22 in the arrow X direction with respect to the grip 14 is prevented. Accordingly, the relative movement of the needle member 18 fixed to the grip 14 and the safety member 22 along the arrow X direction is prevented, and thus the needle tip 36 is not exposed again from the needle protection portion 84 of the safety member 22.

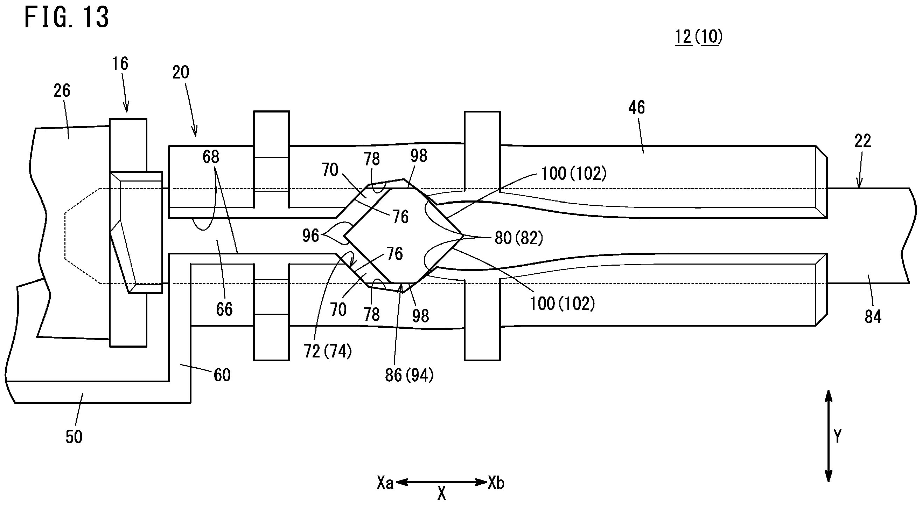

Thereafter, when the grip 14 is further pulled back in the proximal end direction in a state of holding the catheter operation member 20, as illustrated in Fig. 13, while a pair of the second inclined outer surfaces 100 (second restriction portion 102) of the second fitting portion 86 pushes and expands a pair of the second inclined inner surfaces 80 (first restriction portion 82) of the second fitting portion 86 outward in the width direction of the slit 66, a pair of the second inclined outer surfaces 100 (second restriction portion 102) of the second fitting portion 86 passes over the second inclined inner surfaces 80. Thus, since the groove width of the portion of the slit 66 on the proximal end side is wider than the first fitting portion 72, the second fitting portion 86 can be pulled out from the slit 66 in the proximal end direction. Then, when the distal end of the needle protection portion 84 comes out of the connection portion 46, the safety member 22 is separated from the catheter operation member 20.

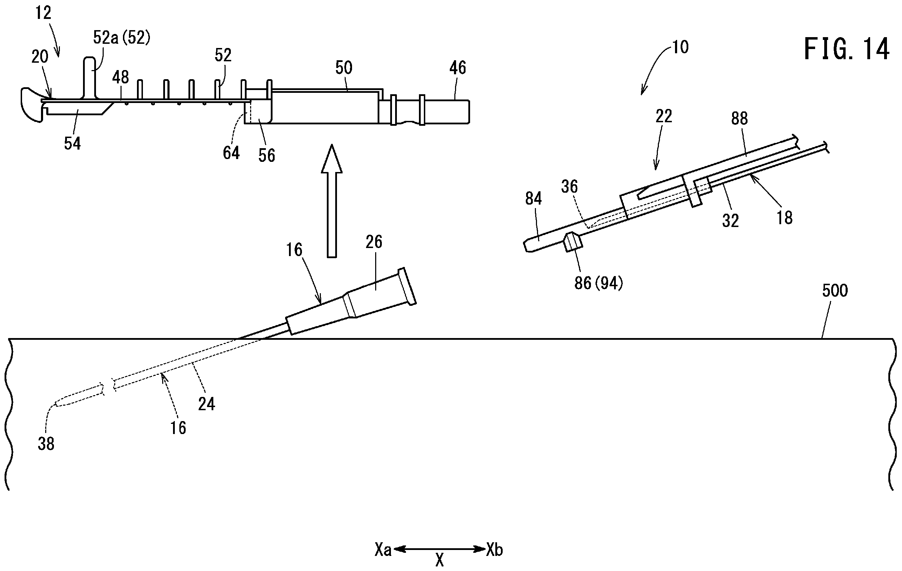

Next, as illustrated in Fig. 14, the catheter operation member 20 is removed from the catheter member 16. Specifically, the catheter 24 is removed from the second support slit 64 by lifting the catheter operation member 20 upward with respect to the catheter member 16.

The present embodiment has the following effects.

According to the present embodiment, when the catheter operation member 20 is moved in the distal end direction to advance the catheter 24 in a state where the living body is punctured with the inner needle 32, the second restriction portion 102 of the safety member 22 can be pushed in the distal end direction by the first restriction portion 82 of the catheter operation member 20. Thus, the safety member 22 can be moved in the distal end direction along with the operation of the catheter operation member 20.

Furthermore, when the safety member 22 is pulled back in the proximal end direction from the catheter operation member 20 after the catheter 24 is advanced, the first restriction portion 82 is pressed in the proximal end direction by the second restriction portion 102, and thus the connection portion 46 can be elastically deformed. Thus, the safety member 22 can be separated from the catheter operation member 20 by causing the second restriction portion 102 to pass over the first restriction portion 82 in the proximal end direction. Therefore, the needle member 18 can be removed from the catheter member 16 while protecting the needle tip 36 by using the safety member 22.

In this case, since the first restriction portion 82 is provided in the connection portion 46 of the catheter operation member 20 and the second restriction portion 102 is provided in the safety member 22, the configurations of the catheter operation member 20 and the safety member 22 can be simplified. Therefore, the size of the catheter assembly 10 can be reduced and the manufacturing cost can be suppressed.

The connection portion 46 has a tubular shape. The slit 66 is formed in the connection portion 46 along the axial direction of the connection portion 46.

According to such a configuration, when the safety member 22 is pulled back in the proximal end direction from the catheter operation member 20, the connection portion 46 is easily elastically deformed radially outward.

The connection portion 46 includes the first fitting portion 72. The safety member 22 includes the needle protection portion 84 inserted into the lumen 47 of the connection portion 46 in the initial state and the second fitting portion 86 provided in the needle protection portion 84 and fitted to the first fitting portion 72 in the initial state. The first restriction portion 82 is provided in the first fitting portion 72. The second restriction portion 102 is provided in the second fitting portion 86.

In such a configuration, the safety member 22 can be easily connected to the connection portion 46 of the catheter operation member 20 by fitting the first fitting portion 72 and the second fitting portion 86 to each other.

The first fitting portion 72 is located on the midway of the connection portion 46 in the axial direction.

In such a configuration, in a case where the safety member 22 is pulled back in the proximal end direction from the catheter operation member 20, the first fitting portion 72 can be brought into contact with the connection portion 46 until the first fitting portion 72 is completely removed from the connection portion 46 after the fitting between the first fitting portion 72 and the second fitting portion 86 is released (the second restriction portion 102 passes over the first restriction portion 82 in the proximal end direction). Thus, it is possible to prevent the safety member 22 from vigorously coming off from the catheter operation member 20 when the fitting between the first fitting portion 72 and the second fitting portion 86 is released.

The first fitting portion 72 is located on the distal end side with respect to the center of the connection portion 46 in the axial direction.

In such a configuration, it is possible to further prevent the safety member 22 from vigorously coming off from the catheter operation member 20 when the fitting between the first fitting portion 72 and the second fitting portion 86 is released.

The first fitting portion 72 is provided on the groove inner surfaces 68 forming the slit 66.

In such a configuration, the first fitting portion 72 can be easily provided in the connection portion 46. Furthermore, it is easy to release the fitting between the first fitting portion 72 and the second fitting portion 86.

The first fitting portion 72 is a fitting hole 74. The second fitting portion 86 is a fitting protrusion 94 that can be fitted into the fitting hole 74.

In such a configuration, since it is not necessary to form a recess in the needle protection portion 84, the thickness of the needle protection portion 84 does not become too thin.

### (Modification example)

Next, an assembly main body 12A according to a modification example will be described. In the present modification example, the same components as those of the above-described assembly main body 12 are denoted by the same reference numerals, and the detailed description thereof will be omitted. In the present modification example, the same effect is obtained for the same configuration as that of the above-described assembly main body 12.

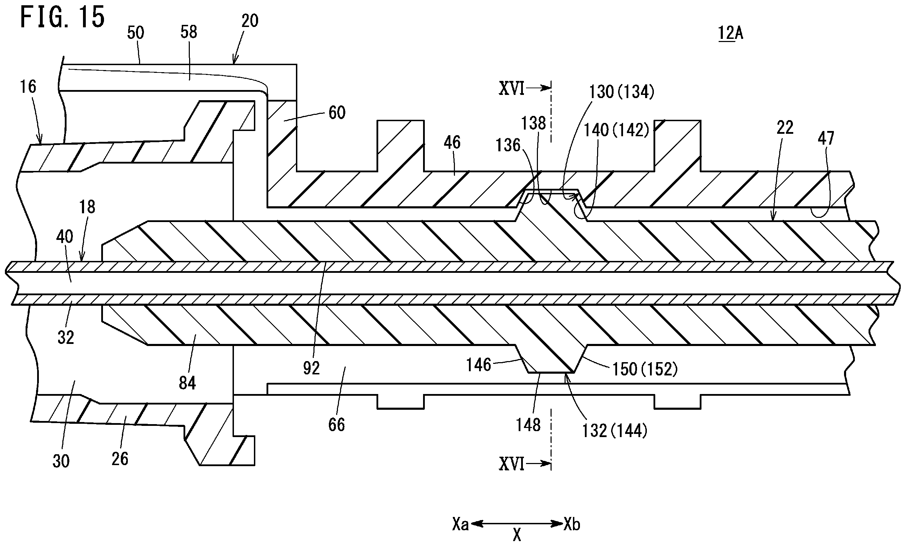

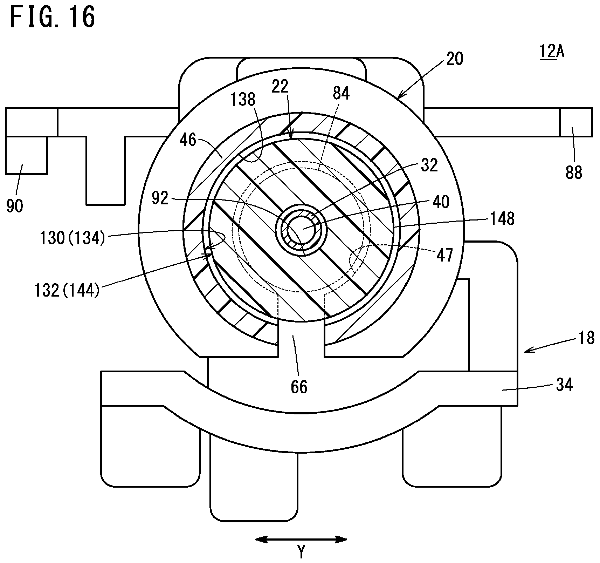

As illustrated in Figs. 15 and 16, in the assembly main body 12A, the structures of a first fitting portion 130 and a second fitting portion 132 are different from the structures of the first fitting portion 72 and the second fitting portion 86 of the assembly main body 12 described above. The first fitting portion 130 is located on the distal end side with respect to the center of the connection portion 46 in the axial direction. The first fitting portion 130 is a fitting recess 134 provided on an inner surface forming the lumen 47 of the connection portion 46. The first fitting portion 130 extends along the circumferential direction of the connection portion 46. Opposite ends of the first fitting portion 130 in the extending direction communicate with the slit 66.

In Fig. 15, the inner surface forming the fitting recess 134 includes a first inclined inner surface 136, an intermediate inner surface 138, and a second inclined inner surface 140. The first inclined inner surface 136 is inclined outward in the radial direction of the connection portion 46 toward the proximal end of the connection portion 46. The intermediate inner surface 138 is connected to an end of the first inclined inner surface 136 in the arrow Xb direction. The intermediate inner surface 138 extends along the axial direction of the connection portion 46.

The second inclined inner surface 140 is connected to an end of the intermediate inner surface 138 in the arrow Xb direction. The second inclined inner surface 140 is inclined inward in the radial direction of the connection portion 46 toward the proximal end of the connection portion 46. The second inclined inner surface 140 is a first restriction portion 142 for restricting the movement of the safety member 22 in the proximal end direction with respect to the connection portion 46.

The second fitting portion 132 is a fitting protrusion 144 protruding from the outer circumferential surface of the needle protection portion 84. The second fitting portion 132 extends along the circumferential direction of the needle protection portion 84. The second fitting portion 132 is inserted into the fitting recess 134 which is the first fitting portion 130 and the slit 66 in the initial state of the catheter assembly 10. The outer surface of the fitting protrusion 144 has a first inclined outer surface 146, an intermediate outer surface 148, and a second inclined outer surface 150.

The first inclined outer surface 146 is inclined outward in the radial direction of the needle protection portion 84 from the end of the second fitting portion 132 in the arrow Xa direction toward the arrow Xb direction. The intermediate outer surfaces 148 is connected to the end of the first inclined outer surface 146 in the arrow Xb direction. The intermediate outer surface 148 extends along the axial direction of the needle protection portion 84. The second inclined outer surface 150 is inclined inward in the radial direction of the connection portion 46 from the proximal end of the intermediate outer surface 148 toward the arrow Xb direction.

In the initial state of the catheter assembly 10, the first inclined outer surface 146 is located to face the first inclined inner surface 136, the intermediate outer surface 148 is located to face the intermediate inner surface 138, and the second inclined outer surface 150 is located to face the second inclined inner surface 140. The second inclined outer surface 150 is a second restriction portion 152 that can come into contact with the second inclined inner surface 140 (first restriction portion 142) in a state where the safety member 22 is connected to the catheter operation member 20. The second restriction portion 152 is located on the distal end side with respect to the first restriction portion 142 in the initial state of the catheter assembly 10.

In the present modification example, the first fitting portion 130 is provided on an inner surface forming the lumen 47 of the connection portion 46.

In such a configuration, as compared with a case where the first fitting portion 130 is provided only on the groove inner surface 68 of the slit 66, the size of the first fitting portion 130 (the contact area between the first restriction portion 142 and the second restriction portion 152) can be easily changed. Thus, it is easy to set the force necessary for separating the safety member 22 from the catheter operation member 20 to an appropriate magnitude.

The second fitting portion 132 extends along the circumferential direction of the needle protection portion 84.

In such a configuration, it is possible to relatively increase the force required for the second restriction portion 152 to pass over the first restriction portion 142.

In the present invention, the first fitting portion may be a protrusion, and the second fitting portion may be a recess. Furthermore, both the first fitting portion and the second fitting portion may be protrusions. The catheter assembly may include a blunt needle that is inserted into the lumen of the inner needle. In this case, since the distal end of the blunt needle can protrude from the distal end of the inner needle before the inner needle is removed from the catheter member, it is possible to prevent the catheter from being damaged by the needle tip when the inner needle is removed.

Note that the present invention is not limited to the above-described embodiment, and various configurations can be taken without departing from the gist of the present invention.

The present embodiment discloses the following content.

According to the embodiment described above, there is disclosed a catheter assembly (10) including: a catheter member (16) including a catheter (24) and a catheter hub (26) provided at a proximal end of the catheter; a needle member (18) including an inner needle (32) inserted into a lumen (28) of the catheter and an inner needle hub (34) provided at a proximal end of the inner needle; a catheter operation member (20) provided in the catheter member; and a safety member (22) provided to be movable with respect to the inner needle in an axial direction of the inner needle, in which a needle tip (36) of the inner needle is exposed from the safety member in an initial state, is located on a distal end side with respect to the safety member, and is covered with the safety member in a state where the inner needle is removed from the catheter member, the catheter operation member includes a connection portion (46) that separably connects the safety member and is formed to be elastically deformable, the connection portion includes a first restriction portion (82, 142), the safety member includes a second restriction portion (102, 152) located on a distal end side with respect to the first restriction portion so as to face the first restriction portion in the initial state, and when the safety member is relatively moved to the catheter operation member in a proximal end direction, the catheter operation member and the safety member are separated from each other since the connection portion pressed by the second restriction portion is elastically deformed and the second restriction portion passes over the first restriction portion in the proximal end direction.

In the catheter assembly, the connection portion may be formed in a tubular shape, and a slit (66) may be formed in the connection portion along an axial direction of the connection portion.

In the catheter assembly, the connection portion may include a first fitting portion (72, 130), the safety member may include a needle protection portion (84) inserted into a lumen (47) of the connection portion in the initial state, and a second fitting portion (86, 132) provided in the needle protection portion and fitted to the first fitting portion in the initial state, the first restriction portion may be provided in the first fitting portion, and the second restriction portion may be provided in the second fitting portion.

In the catheter assembly, the first fitting portion may be located on a midway of the connection portion in the axial direction.

In the catheter assembly, the first fitting portion may be located on the distal end side with respect to a center of the connection portion in the axial direction.

In the catheter assembly, the first fitting portion may be provided on a groove inner surface (68) forming the slit.

In the catheter assembly, the first fitting portion may be provided on an inner surface forming the lumen of the connection portion.

In the catheter assembly, the second fitting portion may annularly extend in a circumferential direction of the needle protection portion.

In the catheter assembly, the first fitting portion may be a recess or a hole, and the second fitting portion may be a protrusion fittable to the recess or the hole.

### Reference Signs List

10 Catheter assembly
16 Catheter member
18 Needle member
20 Catheter operation member
22 Safety member
24 Catheter
26 Catheter hub
32 Inner needle
34 Inner needle hub
36 Needle tip
46 Connection portion
66 Slit
72, 130 First fitting portion
82, 142 First restriction portion
86, 132 Second fitting portion
102, 152 Second restriction portion

## Claims

1. A catheter assembly comprising:
a catheter member including a catheter and a catheter hub provided at a proximal end of the catheter;
a needle member including an inner needle inserted into a lumen of the catheter and an inner needle hub provided at a proximal end of the inner needle;
a catheter operation member provided in the catheter member; and
a safety member provided to be movable with respect to the inner needle in an axial direction of the inner needle,
wherein a needle tip of the inner needle is exposed from the safety member in an initial state, is located on a distal end side with respect to the safety member, and is covered with the safety member in a state where the inner needle is removed from the catheter member,
the catheter operation member includes a connection portion that separably connects the safety member and is formed to be elastically deformable,
the connection portion includes a first restriction portion,
the safety member includes a second restriction portion located on a distal end side with respect to the first restriction portion so as to face the first restriction portion in the initial state, and
when the safety member is relatively moved to the catheter operation member in a proximal end direction, the catheter operation member and the safety member are separated from each other since the connection portion pressed by the second restriction portion is elastically deformed and the second restriction portion passes over the first restriction portion in the proximal end direction.

2. The catheter assembly according to claim 1,
wherein the connection portion is formed in a tubular shape, and
a slit is formed in the connection portion along an axial direction of the connection portion.

3. The catheter assembly according to claim 2,
wherein the connection portion includes a first fitting portion,
the safety member includes
a needle protection portion inserted into a lumen of the connection portion in the initial state, and
a second fitting portion provided in the needle protection portion and fitted to the first fitting portion in the initial state,
the first restriction portion is provided in the first fitting portion, and
the second restriction portion is provided in the second fitting portion.

4. The catheter assembly according to claim 3,
wherein the first fitting portion is located on a midway of the connection portion in the axial direction.

5. The catheter assembly according to claim 4,
wherein the first fitting portion is located on the distal end side with respect to a center of the connection portion in the axial direction.

6. The catheter assembly according to any one of claims 3 to 5,
wherein the first fitting portion is provided on a groove inner surface forming the slit.

7. The catheter assembly according to any one of claims 3 to 5,
wherein the first fitting portion is provided on an inner surface forming the lumen of the connection portion.

8. The catheter assembly according to any one of claims 3 to 7,
wherein the second fitting portion annularly extends in a circumferential direction of the needle protection portion.

9. The catheter assembly according to any one of claims 3 to 8,
wherein the first fitting portion is a recess or a hole, and
the second fitting portion is a protrusion fittable to the recess or the hole.
